# EUROPEAN PATENT SPECIFICATION

(11) **EP 3 089 712 B1**
(45) Date of publication and mention of the grant of the patent: **13.05.2026**
(21) Application number: 14877266.8
(22) Date of filing: 24.12.2014
(51) Int. Cl.: A61F 5/56

(54) **MANDIBULAR ADVANCEMENT DEVICE**
MANDIBULÄRE PROTRUSIONSVORRICHTUNG
DISPOSITIF D'AVANCEMENT MANDIBULAIRE

(30) Priority: 30.12.2013 US 201361922016 P
(43) Date of publication of application: 09.11.2016
(73) Proprietor: Prosomnus Sleep Technologies Inc., Dublin, CA 94568 (US)
(72) Inventor: KUHNS, David W., Dublin, CA 94568 (US); KIM, Sung, Dublin, CA 94568 (US); LIPTAK, Leonard A., Dublin, CA 94568 (US); SHEPPARD, Laura, Dublin, CA 94568 (US)
(74) Representative: FRKelly
(86) International application number: PCT/US2014/072398
(87) International publication number: WO 2015/103084

(56) References cited:
- WO-A1-2009/140720
- WO-A1-2009/140720
- WO-A1-2013/102095
- WO-A1-2013/124452
- WO-A1-2013/124452
- DE-A1- 102004 007 008
- KR-A- 20130 111 848
- RU-C1- 2 491 041
- US-A- 5 611 355
- US-B1- 6 604 527
- US-B2- 7 013 191

## Description

### RELATED APPLICATIONS

### FJELD OF THE INVENTION

The present invention is in the field of mandibular advancement devices.

### BACKGROUND OF THE DISCLOSURE

Snoring and mild sleep apnea are generally thought to be the result of a reduced or partial constriction of the airway during sleep. This may be attributed to soft tissue sinking and applying pressure on the airway during sleep including the mandible dropping and moving backwards. Mandibular advancement devices are designed to move the mandible forward to relieve the force applied from soft tissue during sleep and assisting in opening the airway. Depending on the patient, the advancement of the mandible may vary based on the response of the patient. Optimizing the adjustment is referred to as titration.

Many devices are on the market today that serve to advance the mandible. A dual arch device comprising fins and an adjustment screw block are represented by Somnomed's Somnodent (U.S. Patent No. 6,604,527), the Dynflex Dorsal Appliance, and Dr. Nordstrom's NorSnor II produced by Murdock Labs since the 1980's. Each device has an adjustable upper screw mechanism with block, which upon adjustment applies a force against a lower fin or bite block that then moves the mandible forward. The threaded screw system, requires that the user apply an Allen wrench to adjust the screw for titration. These devices are handmade and built up using polymethylmethacrylate (PMMA) and the corresponding monomer, methylmethacrylate. The device is cured in the dental lab and custom designed for each patient. Reproduction of the quality and accuracy of the device is subject to human skill.

Alternatively, there are devices that make an adjustment by changing out straps of different lengths to titrate the mandible forward. Examples of these are the Silent Nite (U.S. Patent No. 5,365,945) produced by Glidewell, and the Narval (U.S. Patent No. 7,146,982) produced by Resmed. These devices offer a possibly lower profile than the screw adjustment devices and also connect the upper and lower arch together. United States Patent No. 6,604,527 discloses a mandibular advancement device wherein the upper jaw has fitted to it an upper plate and the upper plate is firmly received, and generally comprises a body component and two opposed flange components that are located to be lying in an area beside and close to the posterior teeth, and particularly the buccal side of the upper posterior teeth. PCT Publication No. WO 2013/102095 discloses oral appliances and methods of using oral appliances for treatment of sleep disorders and conditions. PCT Publication No. WO 2013/124452 discloses a method of virtually designing a reduced shape of at least one artificial tooth adapted to be inserted in a denture for a patient. PCT Publication No. WO 2009/140720 discloses an apparatus for the alleviation of sleep apnoea in which dentition engagement units or dental overlays are shaped to engage the maxillary and mandibular dentition of a subject. German Patent Publication No. 10 2004 007008 discloses a stop mechanism attached to jaws of a human patient to keep the airway open for prevention of snoring that has projecting interlocking stops attached to rails fitting over upper and lower rows of teeth. Korean Patent Publication No. 20130111848 discloses a mandible splint with respect to a maxillary that is provided to allow an operator to easily attach the device in an oral cavity of a patient in an examination room and to adjust the device according to symptom or recovery from the symptom of the patient.

Several limitations exist with these existing sleep apnea devices. First, the manual, artisanal fabrication method used by these devices limits the ability to consistently and precisely transfer the prescription into the device design. This limitation results in the need for additional adjustments. Second, the manual artisanal fabrication method requires the layering of monomers, which can subject the patient to higher levels of residual monomers. Third, existing device designs feature multiple components, straps, and screws that decrease durability, reliability, and ease of use. Fourth, the materials used in the manual, artisanal fabrication method require the device to be of a certain size to effectively withstand the normal intraoral forces that the device is subjected to. This size limitation results in decreased patient comfort and patient compliance.

Therefore a need exists to provide a mandibular advancement device that provides advancement of the lower jaw through a series of splints digitally designed and milled to provide accurate increments of advancement for easy titration of the mandible, without the need for an adjustment screw mechanism or adjustment straps.

### SUMMARY OF THE INVENTION

The invention is set out in the appended set of claims.

### BRIEF DESCRIPTION OF THE DRAWINGS

FIG. 1 illustrates an embodiment of the disclosed mandibular advancement devices. FIG. IA shows a front view of the device as it is worn in the mouth, whereas FIG. 1B shows a side view of the same embodiment.
FIG. 2 illustrates an embodiment of the disclosed mandibular advancement devices where the rake angle is set in a neutral orientation. FIG. 2A is an illustration of an embodiment of the upper splint, and FIG. 2B is an illustration of an embodiment of the lower splint.
FIG. 3A illustrates an embodiment of the disclosed mandibular advancement device where the rake angle is set in a recline orientation. FIG. 3B illustrates an embodiment of the disclosed mandibular advancement device where the rake angle is set in a procline orientation.
FIGs. 4A, 4B, 4C, and 4D illustrate several embodiments of the fin design in a predesigned digital library of fins. FIG. 4E illustrates that a fin design from the library is incorporated into an embodiment of the disclosed mandibular advancement device.
FIG 5 illustrates an embodiment of the disclosed mandibular advancement device where there exists a gap between the lower fin and the lower splint.
FIG 6 illustrates varying the plane of the splint with respect to the occlusal plane.
FIG 7 shows the occlusal side of a splint with cutouts designed into the device to accurately place ball clasps.
FIG. 8A shows a free standing upper splint with the retention arms, while FIG. 8B shows the graphics of how the upper splint and the retention arms fit into the mouth of the patient.
FIG. 9 illustrates the titration process using several upper and lower splints.

### DETAILED DESCRIPTION OF THE EMBODIMENTS

In one aspect provided herein is a digitally designed and milled mandibular advancement device comprising an upper splint and a lower splint, wherein the upper and lower splints independently further comprise one or more fins. In some embodiments, the splints provide accurate increments of advancement of the lower jaw for titration of the mandible. The terms "dental splint" and "splint" as used herein refers to several types of orthodontic devices that are designed to address dental problems such as loose teeth and bruxism, in addition to problems with snoring and apnea.

A patient in need of the disclosed mandibular advancement devices wears the upper splint on the upper dentition and the lower splint on the lower dentition during sleep. The splints are designed to remain attached to the dentition until the patient removes them. The fins of the upper and lower splints cause a precise placement of the mandible in relation to the maxilla. The mandible is caused to stay in a forward position and does not relax and fall back. The airway constriction during the sleep is thereby minimized.

In one embodiment the device creates an offset between the upper and lower splint by using upper and lower fins as boundary surfaces to restrict movement while the mouth is closed or reasonably opened.

In some embodiments, the devices disclosed herein were digitally designed and then milled as a single unit. In some of these embodiments, a computer aided design (CAD) process were used to design and manufacture the mandibular advancement devices disclosed herein. A plaster model of the patient's dental impression were first obtained using well-known techniques in the art. Then, scans of the plaster models were imported into the CAD software. In other embodiments, the 3D files of the patient's impression are imported from other sources, such as a direct scan of the patient dentition using an Intra Oral Scan (IOS) Device, e.g., the 3M TruDefTM scanner, or a direct scan of the impression from either an IOS or Cone Beam Computed Tomography (CBCT) device. In these embodiments, the files enable the design of the mandibular advancement splint in 3D space in a CAD software such as 3-Matic by Materialise^{™}.

In other embodiments, the different components of the disclosed devices, for example the splint, the fins, the retention arms, etc., are milled or manufactured separately and then attached together after the manufacturing. This approach allows for the use of interchangeable parts.

In one embodiment the digitally designed and milled splints are reproduced accurately without manual polymer buildup. In some embodiments, accurate reproduction results in accurate replacement devices. In other embodiments, it results in reproducible titration settings. In some embodiments the splints are digitally designed and milled to provide access to pre-cured polymeric materials, and wherein the device has minimal residual monomers.

In one embodiment one or more identifying information, e.g., the patient's name, order number, and other relevant tracking information, are designed into the device. The identifying information appears on the device through the milling process.

In one embodiment, the device is designed to comfortably fit on to a patient's upper and lower arches, maintain a maximum amount of space for the tongue, and keep the mandible advanced forward per a doctor's prescription while still allowing the patient to reasonably open their mouth and move their jaw from left to right for comfort. This contact serves as a barrier to keep the lower fin in a position forward of this fin engagement surface.

The disclosed devices can be made from any material that can withstand the oral environment for an extended period of time, for example overnight. Furthermore, the material can be any material that is capable of being milled to form the devices disclosed herein. Examples of materials include plastics and other polymers, whether hard or soft, transparent or opaque. Some suitable polymers include, but are not limited to, a polyetheretherketone (PEEK), polystyrene, polyvinyl chloride, rubber, synthetic rubber, or an acrylate polymer, such as a polymer made up of methyl methacrylate, methyl acrylate, ethyl acrylate, 2-chloroethyl vinyl ether, 2- ethylhexyl acrylate, hydroxyethyl methacrylate, butyl acrylate, butyl methacrylate, or trimethylolpropane triacrylate (TMPTA).

Embodiments of the device are further described with reference to the accompanying drawings.

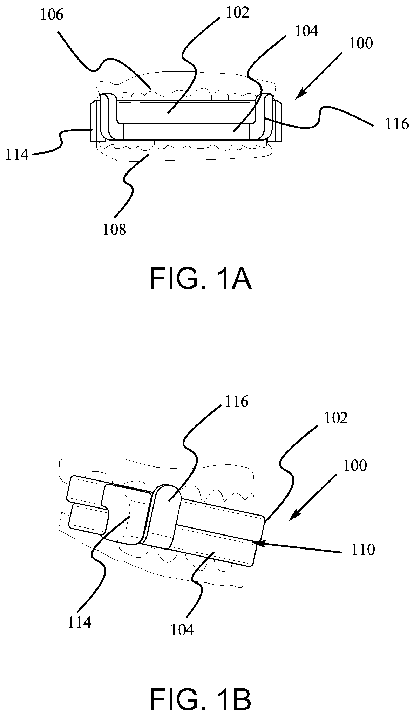

FIG 1 illustrates an embodiment of the disclosed mandibular advancement device, 100. FIG. la shows a front view of the device as it is worn in the mouth, whereas FIG. 1b shows a side view of the same embodiment. The device 100 comprises and upper splint 102 and a lower splint 104. The splint 102 is configured to fit snuggly onto the upper dentition 106, while the lower splint 104 is configured to fit snuggly onto the lower dentition 108. Each splint provides enough retention to keep the device on during normal wear but allowing the user to pull off the device with minimal effort. The two splints contact each other along the occlusal plane 110, i.e., the plane passing through the biting surfaces of the teeth.

In one embodiment the thickness of the upper splint 102 and lower splint 104 is independently varied to create a fixed amount of jaw opening between the patient's arches.

The devices 100 disclosed herein are prepared individually and specially for a particular patient. For this reason, while preparing the digital design of the disclosed devices, the idiosyncrasies of the patient's oral and dentition structures are taken into account. For instance, in some embodiments, the opposing surfaces of the splint are designed in variance with each other to accommodate the patient's oral structure to achieve maximum comfort. The surfaces can be flat, or be made to touch at one or more points. This is true of any other feature of the devices 100. For example, the height, width and shape of the fins; the rake angle; the offset position of the fins; the location of retention arms, if any; the dental impressions; inter alia, are designed specifically for the particular patient. This feature is easily enabled with a device that is digitally pre-designed. The currently available devices are handmade, making it difficult for the artisan to accurately take into account the specific oral features of a particular patient.

Each upper splint 102 comprises at least one upper fin 114, and preferably two upper fins 114. The fin 114 is located to the side of the upper splint 102 such that when the splint 102 is worn by the patient, the fin 114 is near the molars. Each upper fin 114 protrudes downwardly.

Similarly, each lower splint 104 comprises at least one lower fin 116, and preferably two lower fins 116. The fin 116 is located to the side of the lower splint 104 such that when the splint 104 is worn by the patient, the fin 116 is near the molars. Each lower fin 116 protrudes upwardly.

Throughout the present disclosure, the terms "up," "upper," or "upward," and "down," "lower," or "downward" refer to the relative position of the upper jaw and the lower jaw. Thus, "protruding downwardly" means protruding away from the upper jaw and towards the lower jaw. Similarly, the words "front" or "forward" and "back" or "backward" refer to the relative position of components in the mouth. Thus, "front" means towards the lips, whereas "back" means towards the throat, when the device is in the mouth.

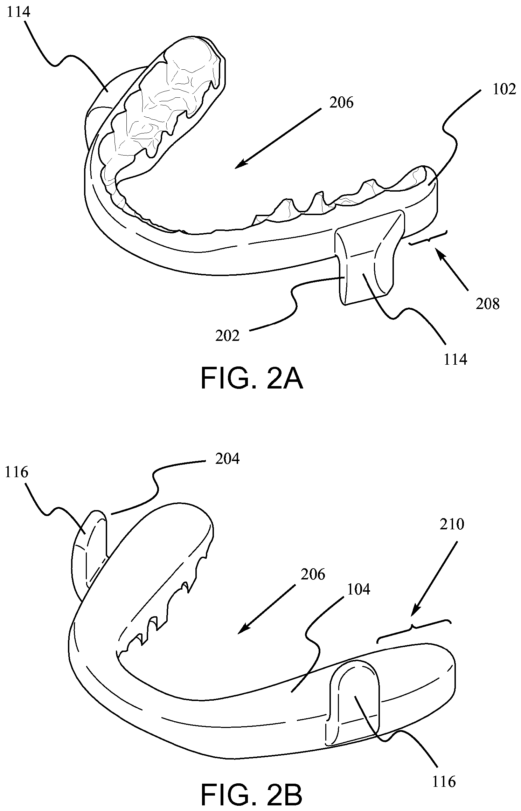

FIG. 2A shows the embodiment of the upper splint that is shown in FIG. 1 and FIG. 2B shows the embodiment of the lower splint that is shown in FIG. 1. FIG. 2 depicts the splints by themselves and without being worn on the teeth.

The upper fin 114 comprises a front surface 202 and the lower fin 116 comprises a back surface 204. When the device is worn in the mouth, the lower fin 116 is located in front of the upper fin 114. The front surface 202 of the upper fin 114 contacts the back surface 204 of the lower fin 116. This contact serves as a barrier to keep the lower fin in a position forward of this fin engagement surface. That is, the contact prevents the lower jaw from moving backward relative to the upper jaw. Thus, once the device is worn, the relative forward position of the upper and lower jaws becomes fixed. The patient would be able to open and close their mouth and move the lower jaw from left to right for comfort. However, the patient would not be able to move the lower jaw backwards beyond the point of contact of the two surfaces 202,204.

In one embodiment the device is digitally designed and milled to provide optimal strength with reduced interference to the tongue creating a comfortable and durable device. As seen in FIGs. 2A & 2B, the two splints each form an arch with an empty center 206. The empty center 206 provides room for the patient's tongue when the device is in use. The type of design that allows room for the tongue is referred to as lingual-less. Thus, some embodiments of the present device 100 are lingual-less designs.

The upper fin 114 is located at a distance UD (208) from the back of the upper splint 102. Similarly, the lower fin 116 is located at a distance LD (210) from the back of the lower splint 104. Distances 208,210 are also referred to as fin offset. The relative positioning of the fins determines the degree to which the lower jaw is protruded forward, i.e., the jaw offset. As discussed below, in some embodiments, the distances 208 and 210 are manipulated to provide the best fit for the patient.

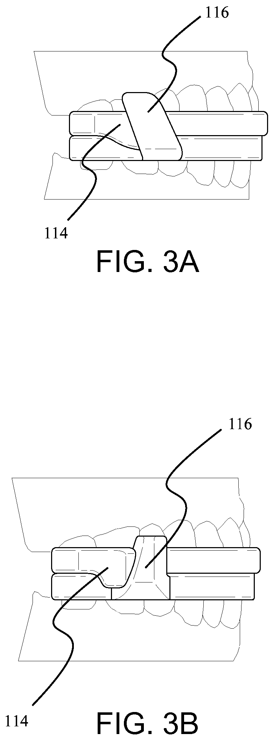

In one embodiment, the upper and lower fins vary in angular shape at the contact surfaces of each fin to provide the most anatomically correct position and comfort during the motion of opening and closing the mouth. The embodiment shown in FIG. 1 comprises vertical contact surfaces. In these embodiments, the contact surfaces 202 and 204 are perpendicular to the occlusal plain 110. In this embodiment, the rake angle, i.e., the angle that surface 202 makes with the occlusal plane 110, is 90° to the occlusal plane, i.e., a neutral angle. In other embodiments, for example those shown in FIGs. 3A and 3B, the contact surfaces are at a non-neutral angle. For example, in the embodiment shown in FIG. 3A, the rake angle is set in a recline orientation. In this embodiment, the surface 202 angles backward. Conversely, in the embodiment shown in FIG. 3B, the rake angle is set in a procline orientation. In this embodiment, the surface 202 angles forward. The rake angle is configured to drive a directional movement during the opening and closing of the mouth. The particular angle, i.e., whether neutral, incline, or procline, and the angle degree, are chosen based on clinician prescription and patient comfort.

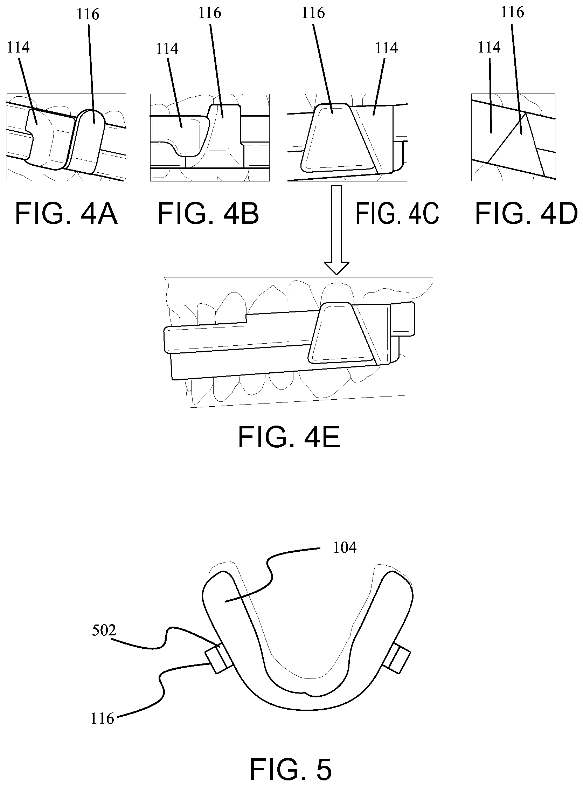

In one embodiment, the fins are selected from a predesigned digital library of fins. FIG. 4 shows additional embodiments of the fin design. FIGs. 4A-4D show some of the embodiments used in a fin library. In some embodiments, a designer calls from a library of fin designs, for example those shown in FIGs. 4A-4D, select one fin type (for example that shown in FIG. 4C), and place onto the custom patient splint design, as shown in FIG. 4E.

As shown in FIG. 5, there exists a gap 502 between the lower fin 116 and the lower splint 104. While the fin 116 is attached to the splint 104 at the base, the fin 116 protrudes slightly outwards before protruding upward. A corresponding gap exists between the upper fin 114 and the upper splint 102. The gap 502 is designed into the device based on the prescription and patient anatomy and comfort. The gap 502 further allows for the side to side motion of the lower jaw with respect to the upper jaw.

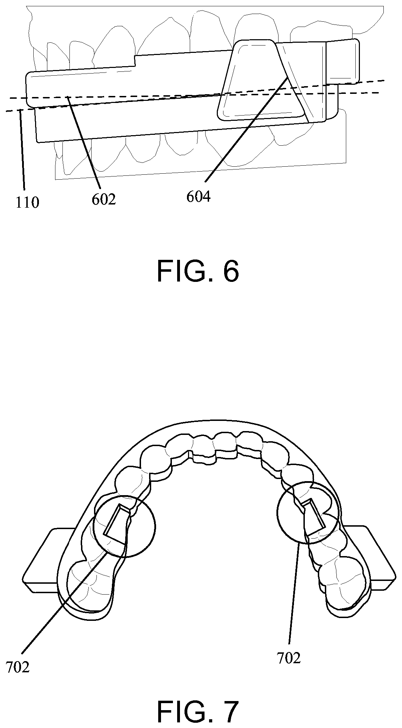

When the device is worn by a patient and the patient's mouth is closed, the bottom surface of the upper splint and the top surface of the lower splint contact each other along the curve of the device, i.e., the arch of the mouth. The two surfaces contact each other along the plane of the splint. In one embodiment the opposing surfaces of the splint are designed such that the plane of the splint equals the occlusal plane. In some embodiments the plane of the splint is at an angle to the occlusal plane. FIG. 6 illustrates the ability to design devices in varying degrees of angle of the plane 110 of the splint to the occlusal plane 602. In CAD, the angle of the plane 110 of the splint can be adjusted within the sagittal plane relative to the patient's anatomical occlusal plane 602. Moreover the angle of the plane 110 can be adjusted within the frontal plane relative to the patient's occlusal plane 602.

Both adjustments are useful to the clinician to affect changes to the relative positioning of the mandible and maxillae. The path of the movement of the upper and lower arches, for the opening and closing of the mouth, is affected by the engagement surface 604, i.e., where the contact surfaces 202 and 204 meet. If the engagement surface 604 is not properly designed, then the upper and lower arches open and close along an unnatural arc, causing pain for the patient. In manufacturing the disclosed devices 100, the natural arc of the patient's jaw movement is taken into account in the digital design. The engagement surface 604 is then designed to fit the natural arc.

In some embodiments, the device 100 is made of transparent materials, for example transparent plastic, to allow the clinician to see the patient's dentition through the device 100 to ensure good fit. Thus, in some embodiments, the clinician can insert a generic device 100 into the patient's mouth and mark the various measurements on the transparent device 100 instead of preparing a dental impression. In other embodiments, a transparent device 100 is prepared first, and relatively inexpensively, and the fit is tested before a more expensive and permanent device is manufactured.

In some embodiments the fins vary in length. In certain embodiments, the fins are adjusted to the patient's open mouth dimensions. In some embodiments, the fins vary in length related to the opposing fin to optimize the length required to maintain mandible offset position while considering comfort such that each fin is the same length or a fraction of the length of the opposing fin. The sum of the heights of an upper fin 114 and lower fin 116 is the total range of contact along the engagement surface before the two splints are separated. At the point of separation, the two splints do not exert pressure on each other and do not provide any jaw offset. In some embodiments, the upper fin 114 and lower fin 116 are of equal heights. In these embodiments, the total range is optimized while minimizing the height of either fin. The ratio of the heights of each fin can also be adjusted for patient comfort or clinical reasons.

In some embodiments, the thickness of each fin can be adjusted to apply more or less pressure against the cheek. The pressure on the cheek stimulates the body to adjust the muscles in the mouth area, e.g., the airway muscles, for patient comfort or clinical reasons.

In one embodiment the device embeds one or more structural features that create strength using less material. In some embodiments the structural feature is made from a single material. The term "embed" as used herein refers to a single material with design geometries or purposefully milled slots or other geometries that enable another material to be added as a support member, in the same way a rebar can strengthen a concrete block. In some embodiments the structural feature is made from a combination of materials, such a metal alloy. In some embodiments the metal or metal alloy is in the shape of a ball clasp, retention wire, or treatment wire. In some embodiments the treatment wires comprise of wires to aid in a clinical result deemed important by the practicing doctor, such as retention wires, alignment wires, or a tongue behavior modification wire such as spikes or barbs to affect tongue thrust.

A patient inserts the disclosed devices 100 into the patient's mouth before sleep and removes them after sleep. The devices should be retained in the mouth snugly enough so that the device does not fall out while the patient is sleeping. However, the fit cannot be too snug so that the patient cannot easily remove the device after waking up. The retention of the device 100 in the mouth is achieved using a combination of one or more of a variety of retention devices and gaps in space between the device and tissue.

In one embodiment the device is retained onto the teeth of a patient using patient specific retention arms. In some embodiments, the retention arms replace or improve the classic use of ball clasps. In the embodiments where ball clasps are used, space for their placement can easily be designed into the splint. In one embodiment, FIG. 7 shows the occlusal side of a splint with cutouts 702 designed into the device to accurately place ball clasps.

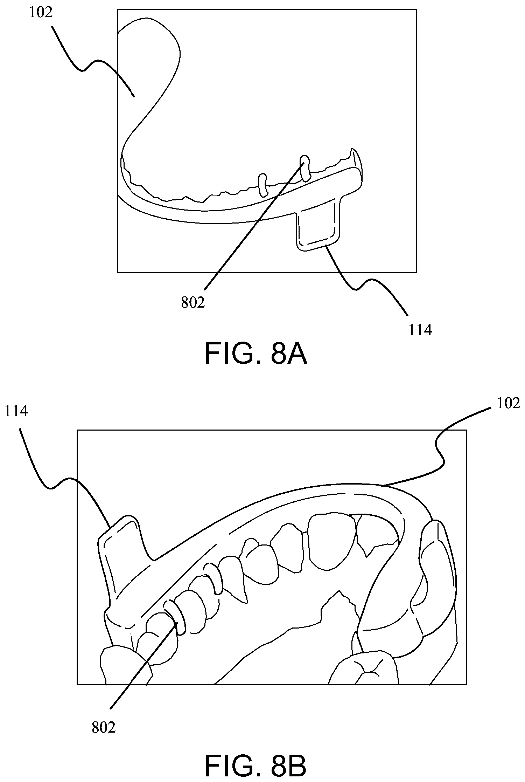

In some embodiments, for example that shown in FIG. 8, the device 100 comprises retention arms 802, built into either or both of the upper splint 102 and lower splint 104. The retention arms 802 allow for a more secure placing of the splints into the mouth. FIG. 8A shows a free standing upper splint 102 with the retention arms 802, while FIG. 8B shows the graphics of how the upper splint 102 and the retention arms fit into the mouth of the patient. In some embodiments, the retention arms 802 take on the shape of the classic ball clasp, while in other embodiments, the retention arms 802 perfectly conform to the patient's anatomy to optimize strength and surface area for retention while allowing for maximum space for the tongue.

In some embodiments, a gap is designed between a particular device surface and one or more surfaces of a patient's anatomy. A purpose of the gaps is to allow the device 100 to be installed easily, stay retained under normal conditions, and be removed easily as well. In some embodiments, the gap is uniform across the entire contact region between the device and the patient's tissue. In other embodiments, the gap is strategically placed with properly designed spacing to provide easy installation of the device in the mouth, optimal device retention onto the patient's dentition, or ease of use of the device. The gap is easily programed into the CAD digital design. Because the disclosed device can be prepared quickly and inexpensively, the clinician or the designer can experiment with a series of different gap placings until the best fit is obtained.

It is possible, through a series of steps, called titration, to choose the device having the most clinically relevant mandibular advancement setting for the patient. Thus, in another aspect, disclosed herein methods of selecting a mandibular advancement device for a patient, the method comprising:
a) obtaining two or more upper splints of the mandibular advancement device, wherein each upper splint comprises one or more upper fins, wherein each upper fin is located at a distance UD from back of the upper splint, and wherein the distance UD of any of the two or more upper splints is different than the distance UD of any other of the two or more upper splints; and
   obtaining two or more lower splints of the mandibular advancement device, wherein each lower splint comprises one or more lower fins, wherein each lower fin is located at a distance LD from back of the lower splint, and wherein the distance LD of any of the two or more lower splints is different than the distance LD of any other of the two or more lower splints;
b) choosing a combination of one upper splint and one lower splint for the patient;
c) observing the clinical outcome of the chosen combination of one upper splint and one lower splint;
d) choosing a different combination of one upper splint and one lower splint for the patient if the clinical outcome of step c) is unacceptable; and
e) repeating steps b) - d) until an acceptable clinical outcome is obtained.

Once a digital scan of the patient's dentition is obtained, a number of upper and lower splints are milled for the patient. Each of the upper and lower splints has a different fin offset setting. The clinician chooses one set of upper and lower splints for the patient. If the patient's condition is not improved sufficiently, the clinician then chooses another set of splints. This process is continued until a set of splints providing the best clinical outcome is chosen. An advantage of the devices and methods disclosed herein is that by digitally designing the splints and automatedly manufacturing them, several splints can be prepared relatively quickly and inexpensively. Further, the digital design allows for a precise positioning of the fins. A more effective mandibular advancement can then be obtained than by using a hand-milled and hand-cranked device.

In some embodiments, the titration settings provide for a flexible positioning of the fins in the mouth in the mesial-distal direction. In some embodiments, the fit of the device 100 on a patient is titrated through the use of devices 100 with varying distances 208 and 210 (see FIG. 2). In certain embodiments, the device 100 is milled directly from a CAD file such that the accuracy of the data from the impression of the patient's anatomy, the fit of the device to that data, and the design of the device are precisely transferred to the milling machine. This enables very precise design adjustment in positioning each of the fins in the splint, i.e., the measurement of the distances 208 and 210, and in their position relative to each other in a reproducible and manufacturable way.

For example, in one embodiment, three different upper splints 102 were manufactured having increasingly longer distances 208, i.e., longer fin offset. Also, two different lower splints 104 were manufactured having two different distances 210. Thus, pairing one lower splint 104 with one upper splint 102 provided one jaw offset, while pairing the same upper splint 102 with a different lower splint 104 provided another jaw offset, and so on.

An illustrative example is shown in Table 1. To compile this table, five different upper splints 102 were prepared having 0.0 mm, 1.0 mm, 2.0 mm, 3.0 mm, and 4.0 mm fin offset (distance 208), respectively. Also, four different lower splints 104 were prepared having 0.0 mm, 0.5 mm, 1.0 mm, and 2.0 mm fin offset (distance 210), respectively. The combination of the two different splints can provide a jaw offset ranging from 0.0 mm to 6.0 mm, as shown in Table 1, where U-1 to U-5 are the upper splint offsets (shown in parentheses) and L-A to L-D are the lower splint offsets (shown in parentheses).

**Table 1**

| | U-1 (0.0 mm) | U-2 (1.0 mm) | U-3 (2.0 mm) | U-4 (3.0 mm) | U-5 (4.0 mm) |
|---|---|---|---|---|---|
| L-A (0.0 mm) | 0.0mm | 1.0mm | 2.0mm | 3.0mm | 4.0mm |
| L-B (0.5 mm) | 0.5mm | 1.5 mm | 2.5mm | 3.5mm | 4.5mm |
| L-C (1.0 mm) | 1.0mm | 2.0mm | 3.0mm | 4.0mm | 5.0mm |
| L-D (2.0 mm) | 2.0mm | 3.0mm | 4.0mm | 5.0mm | 6.0mm |

Thus, twenty different mandibular advancements can be obtained with only nine different splints, four lower splints and five upper splints.

Depending on the clinician's prescription need, the clinician chooses any reasonable value for the offset of the upper fin relative to the lower fin, and as many offsets as the clinician desires. Since the digital manufacturing process accurately and precisely reproduces the splints, the combination of splints is repeatable, regardless of when the clinician orders several splints or splints with other offset distances.

Another advantage of the disclosed splint combination is enabling the creation of the same offset with different combinations of upper and lower splint positions. For example, as shown in Table 1, a 3.0 mm offset may be created using three different combinations of upper and lower fins (L-A/U-4, L-C/U-3, and L-D/U-2). Varying the combined position of the two fins allow better alignment of the fins within the mouth for reasons of patient comfort and clinical requirements.

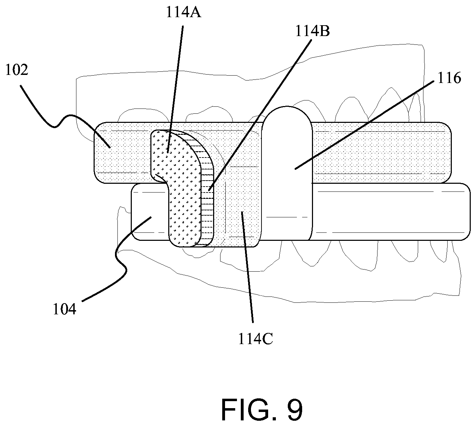

FIG. 9 illustrates the titration. Three separate upper splints 102 are provided, one having an upper fin offset of 1 mm (114-A), one having an upper fin offset of 2 mm (114-B), and one having an upper fin offset of 3 mm (114-B) (FIG. 9 shows all three of these upper splints superimposed on each other for illustration purposes. In actuality, they are separate splints.) One lower splint 104, having a lower fin offset of 0.5 mm (116) is also provided. Not shown is a lower splint 104 having a lower fin offset of O mm. The following combinations of splints provide the mandibular advancements of Table 2.

**Table 2**

| | | | |
|---|---|---|---|
| Upper ► | 1.0 mm | .0mm | 3.0mm |
| Lower ▼ | | | |
| 0.0mm | 1.0mm | 2.0mm | 3.0mm |
| 0.5mm | 1.5 mm | 2.5mm | 3.5mm |

In another aspect, disclosed herein are methods of reducing partial constriction of airway during sleep for a patient, the method comprising identifying a patient in need thereof; and administering to the patient the mandibular advancement device as disclosed herein.

In another aspect, disclosed herein are methods of manufacturing a mandibular advancement device, the method comprising obtaining measurements from a patient's dentition; digitally designing a mandibular advancement device; and milling the mandibular advancement device. In some embodiments, the obtaining measurement step comprises obtaining a dental impression.

## Claims

1. A mandibular advancement device (100) for a patient comprising:
an upper splint (102) and a lower splint (104);
wherein the upper splint (102) comprises at least one upper fin (114), wherein the upper fin is located at a distance UD from back of the upper splint;
and the lower splint (104) comprises at least one lower fin (116), wherein the lower fin is located at a first distance LD from back of the lower splint;
and the upper splint comprises an imprint of an upper dentition of the patient configured to fit onto the upper dentition, and the lower splint comprises an imprint of a lower dentition of the patient configured to fit onto the lower dentition,
and the at least one upper fin is affixed to the upper splint buccal to the buccal surface of the maxillary teeth, and when the upper splint is fit onto the upper dentition the at least one upper fin protrudes downward below the occlusal plane;
and the at least one lower fin is affixed to the lower splint buccal to the buccal surface of the mandibular teeth, and when the lower splint is fit onto the lower dentition the at least one lower fin protrudes upward above the occlusal plane;
each splint independently comprises a flat surface along a plane of the splint away from the patient's dentition;
each fin is "L" shaped, with the leg of the "L" connecting the fin to its respective splint protruding along the occlusal plane buccally, while the other leg of the "L" protruding perpendicular to the occlusal plane away from the patient's gingiva to provide a gap (502) between the upper fin (114) and the upper splint (102) based on the patient anatomy;
each splint independently forms an arch with an empty centre (206); each splint and its respective fins comprise a single contiguous unit; wherein each distance UD and each distance LD is unchangeable;
wherein each of the at least one upper fin (114) comprises a front surface (202) and each of the at least one lower fin (116) comprises a back surface (204), wherein when the upper (102) and lower (104) splints are worn by the patient and the mouth is closed or reasonably closed, the front surface (202) of the at least one upper fin (114) contacts the back surface (204) of the at least one lower fin (116) along a contact surface, thereby causing mandibular advancement of a lower jaw of the patient; and
wherein each upper fin and each lower fin are of sufficient height such that when the patient's mouth is opened during sleep, the contact between the front surface of the upper fin and the back surface of the lower fin is maintained.

2. The device of claim 1, wherein the empty centre (206) provides room for a patient's tongue when the device is in use.

3. The device of claim 1 or 2, wherein a rake angle of the contact surface is selected from the group consisting of a neutral angle, a recline angle, and a procline angle.

4. The device of any one of claims 1 to 3, wherein when an individual wearing the device closes his or her mouth the upper splint (102) contacts the lower splint (104) along a plane, wherein the plane is the same as a patient's occlusal plane.

5. The device of any one of claims 1 to 4, further comprising one or more structural feature configured to accommodate an add on selected from the group consisting of a support member, a ball clasp, retention wire, treatment wire, alignment wires, and a tongue behavior modification wire.

6. The device of any one of claims 1 to 5, further comprising one or more retention arms.

7. The device of any one of claims 1 to 6, wherein when the device is worn by a patient and the patient's mouth is closed, the bottom surface of the upper splint and the top surface of the lower splint contact each other along the curve of the device, or the arch of the mouth, and the upper and lower splint contact each other along the plane of the splint, and the opposing surfaces of the splint are designed such that the plane of the splint equals the occlusal plane.

8. A method of manufacturing a mandibular advancement device as set forth in claim 1, the method comprising the steps of:
obtaining electronic data on the shape of an individual's dentition;
digitally designing a mandibular advancement device according to the obtained data, wherein the mandibular advancement device comprises an upper splint (102) and a lower splint (104);
automatedly milling each of the upper and lower splints from a single polymeric block according to the digital design.

9. The method of claim 8, wherein each of the upper splint (102) and lower (104) splint is designed digitally and milled as a single unit according to a digital design.

10. The method of claims 8 or 9, wherein the shape of each upper fin (114) and each lower fin (116) is independently selected from a predesigned digital library of fins.

11. The method of claim 8, further comprising including in the digital design one or two or more gaps (502) between: the at least one lower fin (116) and the lower splint (104), or the upper fin (114) and the upper splint, wherein the gaps (502) are between the patient's dentition and each of the lower splint (104) or upper splint (102), and optionally wherein the gaps (502) are digitally designed to be varied in size, wherein the varied-sized gaps (502) optimize the retention of the device on the patient's dentition.

## Patentansprüche

1. Unterkieferprotrusionsvorrichtung (100) für einen Patienten, umfassend:
eine obere Schiene (102) und eine untere Schiene (104);
wobei die obere Schiene (102) mindestens eine obere Finne (114) umfasst, wobei die obere Finne in einem Abstand UD von der Rückseite der oberen Schiene angeordnet ist;
und die untere Schiene (104) mindestens eine untere Finne (116) umfasst, wobei die untere Finne in einem ersten Abstand LD von der Rückseite der unteren Schiene angeordnet ist;
und die obere Schiene einen Abdruck eines oberen Gebisses des Patienten umfasst, der so konfiguriert ist, dass er auf das obere Gebiss passt, und die untere Schiene einen Abdruck eines unteren Gebisses des Patienten umfasst, der so konfiguriert ist, dass er auf das untere Gebiss passt,
und die mindestens eine obere Finne an der oberen Schiene bukkal zur Bukkalfläche der Oberkieferzähne befestigt ist, und wenn die obere Schiene auf das obere Gebiss aufgesetzt ist, die mindestens eine obere Finne nach unten unter die Okklusionsebene ragt;
und die mindestens eine untere Finne an der unteren Schiene bukkal zur Bukkalfläche der Unterkieferzähne befestigt ist, und wenn die untere Schiene auf das untere Gebiss aufgesetzt ist, die mindestens eine untere Finne nach oben über die Okklusionsebene ragt;
jede Schiene unabhängig eine ebene Fläche entlang einer Ebene der Schiene weg vom Gebiss des Patienten umfasst;
jede Finne L-förmig ist, wobei der Schenkel des "L", der die Finne mit der jeweiligen Schiene verbindet, entlang der Okklusionsebene bukkal ragt, während der andere Schenkel des "L" senkrecht zur Okklusionsebene weg von der Gingiva des Patienten ragt, um einen Spalt (502) zwischen der unteren Finne (114) und der oberen Schiene (102) basierend auf der Patientenanatomie bereitzustellen;
jede Schiene unabhängig einen Bogen mit einem leeren Zentrum (206) bildet; jede Schiene und ihre jeweiligen Finnen eine einzelne zusammenhängende Einheit umfassen; wobei jeder Abstand UD und jeder Abstand LD unveränderlich ist;
wobei jede der mindestens einen oberen Finne (114) eine Vorderfläche (202) umfasst und jede der mindestens einen unteren Finne (116) eine Rückfläche (204) umfasst, wobei, wenn die obere (102) und untere (104) Schiene vom Patienten getragen werden und der Mund geschlossen oder im Wesentlichen geschlossen ist, die Vorderfläche (202) der mindestens einen oberen Finne (114) die Rückfläche (204) der mindestens einen unteren Finne (116) entlang einer Kontaktfläche berührt, wodurch ein Unterkiefervorschub des Unterkiefers des Patienten bewirkt wird; und
wobei jede obere Finne und jede untere Finne eine ausreichende Höhe aufweisen, so dass, wenn der Mund des Patienten während des Schlafes geöffnet wird, der Kontakt zwischen der Vorderfläche der oberen Finne und der Rückfläche der unteren Finne aufrechterhalten wird.

2. Vorrichtung nach Anspruch 1, wobei das leere Zentrum (206) Platz für die Zunge des Patienten bereitstellt, wenn die Vorrichtung in Gebrauch ist.

3. Vorrichtung nach Anspruch 1 oder 2, wobei ein Neigungswinkel der Kontaktfläche ausgewählt ist aus der Gruppe bestehend aus einem neutralen Winkel, einem Reklinationswinkel und einem Proklinationswinkel.

4. Vorrichtung nach einem der Ansprüche 1 bis 3, wobei, wenn eine Person, die die Vorrichtung trägt, ihren Mund schließt, die obere Schiene (102) die untere Schiene (104) entlang einer Ebene berührt, wobei die Ebene dieselbe ist wie die Okklusionsebene des Patienten.

5. Vorrichtung nach einem der Ansprüche 1 bis 4, ferner umfassend ein oder mehrere strukturelle Merkmale, die zur Aufnahme eines Zusatzteils konfiguriert sind, das ausgewählt ist aus der Gruppe bestehend aus einem Stützelement, einer Kugelklammer, einem Retentionsdraht, einem Behandlungsdraht, Ausrichtungsdrähten und einem Zungenverhaltensmodifikationsdraht.

6. Vorrichtung nach einem der Ansprüche 1 bis 5, ferner umfassend einen oder mehrere Retentionsarme.

7. Vorrichtung nach einem der Ansprüche 1 bis 6, wobei, wenn die Vorrichtung von einem Patienten getragen wird und der Mund des Patienten geschlossen ist, die Unterseite der oberen Schiene und die Oberseite der unteren Schiene einander entlang der Krümmung der Vorrichtung oder des Bogens des Mundes berühren und die obere und untere Schiene einander entlang der Ebene der Schiene berühren und die gegenüberliegenden Flächen der Schiene so gestaltet sind, dass die Ebene der Schiene gleich der Okklusionsebene ist.

8. Verfahren zur Herstellung einer Unterkieferprotrusionsvorrichtung nach Anspruch 1, wobei das Verfahren die folgenden Schritte umfasst:
Erfassen elektronischer Daten über die Form des Gebisses einer Person;
digitales Entwerfen einer Unterkieferprotrusionsvorrichtung gemäß den erfassten Daten, wobei die Unterkieferprotrusionsvorrichtung eine obere Schiene (102) und eine untere Schiene (104) umfasst;
automatisiertes Fräsen jeder der oberen und unteren Schienen aus einem einzelnen Polymerblock gemäß dem digitalen Entwurf.

9. Verfahren nach Anspruch 8, wobei jede der oberen Schiene (102) und unteren (104) Schiene digital entworfen und als einzelne Einheit gemäß einem digitalen Entwurf gefräst wird.

10. Verfahren nach Anspruch 8 oder 9, wobei die Form jeder oberen Finne (114) und jeder unteren Finne (116) unabhängig aus einer vorentworfenen digitalen Bibliothek von Finnen ausgewählt wird.

11. Verfahren nach Anspruch 8, ferner umfassend das Einbeziehen eines oder zweier oder mehrerer Spalte (502) in den digitalen Entwurf zwischen: der mindestens einen unteren Finne (116) und der unteren Schiene (104), oder der oberen Finne (114) und der oberen Schiene, wobei die Spalte (502) zwischen dem Gebiss des Patienten und jeder der unteren Schiene (104) oder oberen Schiene (102) liegen, und wobei optional die Spalte (502) digital so entworfen sind, dass sie in der Größe variiert sind, wobei die in der Größe variierten Spalte (502) die Retention der Vorrichtung am Gebiss des Patienten optimieren.

## Revendications

1. Dispositif d'avancement mandibulaire (100) destiné à un patient, comprenant :
une gouttière supérieure (102) et une gouttière inférieure (104) ;
dans lequel la gouttière supérieure (102) comprend une ailette supérieure (114), l'ailette supérieure étant située à une distance UD par rapport à l'arrière de la gouttière supérieure ;
et la gouttière inférieure (104) comprend une ailette inférieure (116), l'ailette inférieure étant située à une première distance LD par rapport à l'arrière de la gouttière inférieure ;
et la gouttière supérieure comprend une empreinte d'une dentition supérieure du patient, configurée pour s'adapter à la dentition supérieure, et la gouttière inférieure comprend une empreinte d'une dentition inférieure du patient, configurée pour s'adapter à la dentition inférieure ;
et l'ailette supérieure est fixée à la gouttière supérieure, en position buccale par rapport à la surface buccale des dents maxillaires, et, lorsque la gouttière supérieure est adaptée à la dentition supérieure, l'ailette supérieure fait saillie vers le bas, au-dessous du plan occlusal ;
et l'ailette inférieure est fixée à la gouttière inférieure, en position buccale par rapport à la surface buccale des dents mandibulaires, et, lorsque la gouttière inférieure est adaptée à la dentition inférieure, l'ailette inférieure fait saillie vers le haut, au-dessus du plan occlusal ;
chaque gouttière comprend, indépendamment l'une de l'autre, une surface plane située dans le plan de la gouttière, à l'écart de la dentition du patient ;
chaque ailette présente une forme en « L », la branche du « L » reliant l'ailette à sa gouttière respective faisant saillie le long du plan occlusal du côté vestibulaire, tandis que l'autre branche du « L » fait saillie perpendiculairement au plan occlusal, à l'écart de la gencive du patient afin de ménager un espace (502) entre l'ailette supérieure (114) et la gouttière supérieure (102), en fonction de l'anatomie du patient ;
chaque gouttière forme, indépendamment l'une de l'autre, une arcade dotée d'un centre vide (206) ; chaque gouttière et ses ailettes respectives constituent une unité unique et contiguë, les distances UD et LD étant invariables ;
dans lequel chacune des ailettes supérieures (114) comprend une surface avant (202) et chacune des ailettes inférieures (116) comprend une surface arrière (204), de sorte que, lorsque les gouttières supérieure (102) et inférieure (104) sont portées par le patient et que la bouche est fermée ou sensiblement fermée, la surface avant (202) de l'ailette supérieure (114) entre en contact avec la surface arrière (204) de l'ailette inférieure (116) le long d'une surface de contact, provoquant ainsi un avancement mandibulaire de la mâchoire inférieure du patient ; et
dans lequel chaque ailette supérieure et chaque ailette inférieure sont d'une hauteur suffisante pour que, lorsque la bouche du patient s'ouvre pendant le sommeil, le contact entre la surface avant de l'ailette supérieure et la surface arrière de l'ailette inférieure soit maintenu.

2. Dispositif selon la revendication 1, dans lequel le centre vide (206) ménage un espace pour la langue du patient lorsque le dispositif est en cours d'utilisation.

3. Dispositif selon la revendication 1 ou 2, dans lequel un angle d'inclinaison de la surface de contact est sélectionné dans le groupe constitué d'un angle neutre, d'un angle incliné vers l'arrière et d'un angle incliné vers l'avant.

4. Dispositif selon l'une quelconque des revendications 1 à 3, dans lequel, lorsqu'un individu portant le dispositif ferme la bouche, la gouttière supérieure (102) entre en contact avec la gouttière inférieure (104) le long d'un plan, ledit plan étant identique au plan occlusal du patient.

5. Dispositif selon l'une quelconque des revendications 1 à 4, comprenant en outre une ou plusieurs caractéristiques structurelles configurées pour recevoir un élément complémentaire sélectionné dans le groupe constitué d'un élément de support, d'un fermoir boule, d'un fil de rétention, d'un fil de traitement, de fils d'alignement et d'un fil de modification du comportement lingual.

6. Dispositif selon l'une quelconque des revendications 1 à 5, comprenant en outre un ou plusieurs bras de rétention.

7. Dispositif selon l'une quelconque des revendications 1 à 6, dans lequel, lorsque le dispositif est porté par un patient et que la bouche du patient est fermée, la surface inférieure de la gouttière supérieure et la surface supérieure de la gouttière inférieure entrent en contact l'une avec l'autre le long de la courbure du dispositif, ou de la voûte buccale, et les gouttière supérieure et inférieure entrent en contact l'une avec l'autre le long du plan de la gouttière, les surfaces opposées des gouttières étant conçues pour que le plan de la gouttière coïncide avec le plan occlusal.

8. Procédé de fabrication d'un dispositif d'avancement mandibulaire tel que défini dans la revendication 1, le procédé comprenant les étapes consistant à :
obtenir des données électroniques relatives à la forme de la dentition d'un individu ;
concevoir numériquement un dispositif d'avancement mandibulaire conformément aux données obtenues, ledit dispositif d'avancement mandibulaire comprenant une gouttière supérieure (102) et une gouttière inférieure (104) ;
usiner automatiquement chacune des gouttières supérieure et inférieure à partir d'un bloc polymère unique, conformément à la conception numérique.

9. Procédé selon la revendication 8, dans lequel chacune des gouttières supérieure (102) et inférieure (104) est conçue numériquement et usinée en une seule pièce, conformément à une conception numérique.

10. Procédé selon les revendications 8 ou 9, dans lequel la forme de chaque ailette supérieure (114) et de chaque ailette inférieure (116) est sélectionnée indépendamment à partir d'une bibliothèque numérique d'ailettes préconçues.

11. Procédé selon la revendication 8, comprenant en outre l'inclusion, dans la conception numérique, d'un, de deux ou de plusieurs espaces (502) situés entre : l'ailette inférieure (116) et la gouttière inférieure (104), ou l'ailette supérieure (114) et la gouttière supérieure, lesdits espaces (502) étant situés entre la dentition du patient et chacune des gouttières inférieure (104) ou supérieure (102), et, facultativement, lesdits espaces (502) étant conçus numériquement de manière à présenter des dimensions variables, lesdits espaces (502) de dimensions variables optimisant la rétention du dispositif sur la dentition du patient.
